(19) Europäisches Patentamt
European Patent Office
Office européen des brevets

(11) Veröffentlichungsnummer : **0 044 278**
**B1**

(12) **EUROPÄISCHE PATENTSCHRIFT**

(45) Veröffentlichungstag der Patentschrift : **26.10.83**

(51) Int. Cl.³ : **C 07 C127/22**, A 01 N 47/34

(21) Anmeldenummer : **81810279.0**

(22) Anmeldetag : **10.07.81**

(54) **Phenylharnstoffe.**

(30) Priorität : **16.07.80 CH 5457/80**
**12.08.80 CH 6095/80**
**26.02.81 CH 1294/81**
**03.06.81 CH 3628/81**

(43) Veröffentlichungstag der Anmeldung : **20.01.82 Patentblatt 82/03**

(45) Bekanntmachung des Hinweises auf die Patenterteilung : **26.10.83 Patentblatt 83/43**

(84) Benannte Vertragsstaaten :
**BE CH DE FR GB IT LI NL**

(56) Entgegenhaltungen :
**JOURNAL OF AGRICULTURAL AND FOOD CHEMISTRY, Band 21, Nr. 3, Mai/Juni 1973, Seiten 348-354 American Chemical Society Washington D.C., U.S.A. K. WELLINGA et al. : « Synthesis and laboratory evaluation of 1-(2,6-disubstituted benzoyl)-3-phenyl-ureas, a new class of insecticides. I. 1-(2,6-dichlorobenzoyl)-3-phenylureas »**

(73) Patentinhaber : **CIBA-GEIGY AG**
**Patentabteilung Postfach**
**CH-4002 Basel (CH)**

(72) Erfinder : **Ehrenfreund, Josef, Dr.**
**Langenhagweg 11**
**CH-4123 Allschwil (CH)**

**EP 0 044 278 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Phenylharnstoffe

Die vorliegende Erfindung betrifft neue substituierte N-(p-Aminophenyl)-N'-benzoylharnstoffe, Verfahren zu ihrer Herstellung und ihre Verwendung in der Schädlingsbekämpfung.

Die erfindungsgemässen substituierten N-(p-Aminophenyl)-N'-benzoylharnstoffe haben die Formel I

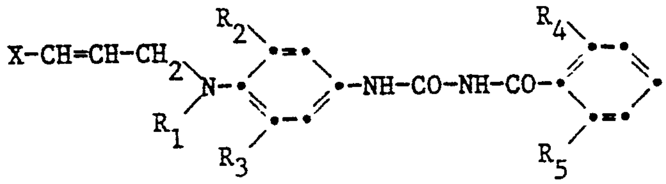

(I)

worin $R_1$ Wasserstoff, $C_1$-$C_4$-Alkyl, $C_3$-Alkenyl, mit einem Chlor- oder Bromatom substituiertes $C_3$-Alkenyl oder $C_3$-$C_6$-Cycloalkyl ; $R_2$ und $R_3$ Wasserstoff oder Halogen ; $R_4$ Methyl oder Halogen, $R_5$ Wasserstoff oder Halogen und X Chlor oder Brom bedeuten.

Gegenstand der Erfindung bilden auch die pflanzenphysiologisch unbedenklichen und landwirtschaftlich geeigneten Salze der Verbindungen der Formel I. Beispiele für zur Salzbildung geeignete Säuren sind Halogenwasserstoffsäuren, wie HCl, HBr, HJ, Phosphorsäure, Perchlorsäure, Rhodanwasserstoffsäure, Tetrafluorborsäure, Salpetersäure, Schwefelsäure, aliphatische und aromatische Sulfonsäuren, Fettsäuren, Trichlor- und Trifluoressigsäure sowie mehrwertige organische Säuren, wie Oxalsäure, Malonsäure, Bernsteinsäure, Weinsäure, Adipinsäure und Zitronensäure.

Beispiele für Alkyl-Gruppen in der Definition $C_1$-$C_4$-Alkyl gemäss der Erfindung sind Methyl, Aethyl, n-Propyl, i-Propyl sowie die vier isomeren Butyl-Gruppen. Im Rahmen der Erfindung ist ferner unter Halogen Fluor, Chlor, Brom und Jod, vorzugsweise Fluor, Chlor und Brom, zu verstehen.

Die Verbindungen der Formel I fallen im allgemeinen als cis-trans-Isomerengemische an. In diesem Sinne sind als Verbindungen der Formel I sowohl cis- oder trans-Formen als auch die entsprechenden Isomerengemische bzw. Isomerenkombinationen zu verstehen. Ein Isomerengemisch kann z. B. mit Hilfe bekannter chromatographischer Trennmethoden in die isomeren Formen getrennt werden. In gewissen Fällen kann die Isomerentrennung auch durch fraktionierte Kristallisation erfolgen.

Wegen ihrer Wirkung als Schädlingsbekämpfungsmittel bevorzugt sind Verbindungen der Formel I, die dadurch gekennzeichnet sind, dass $R_1$ $C_1$-$C_4$-Alkyl, $CH_2=CH—CH_2—$, $Cl—CH=CH—CH_2—$, $Br—CH=CH—CH_2$ oder Cyclopropyl ; $R_2$ und $R_3$ Chlor oder Brom ; $R_4$ Fluor, Chlor, Brom oder Methyl und $R_5$ Wasserstoff, Fluor oder Chlor bedeuten. Von Bedeutung sind daneben solche Verbindungen der Formel I, worin $R_1$ $C_1$-$C_4$-Alkyl ; $R_2$ und $R_3$ Chlor oder Brom ; $R_4$ Fluor oder Chlor und $R_5$ Wasserstoff, Fluor oder Chlor bedeuten. Hervorzuheben sind auch die Verbindungen der Formel I, worin $R_2$ und $R_3$ Chlor bedeuten. Wertvoll sind aufgrund ihrer biologischen Wirksamkeit ferner diejenigen Verbindungen der Formel I, worin $R_1$ Methyl bedeutet, sowie diejenigen, worin $R_4$ und $R_5$ Fluor oder Chlor und diejenigen, worin $R_4$ und $R_5$ Fluor bedeuten.

Die Verbindungen der Formel I können nach an sich bekannten Verfahren hergestellt werden (vgl. u. a. die deutschen Offenlegungsschriften Nr. 2.123.236, 2.601.780 und die europäische Patentanmeldung 13 414).

So kann man z. B. eine Verbindung der Formel I erhalten durch Umsetzung

a) einer Verbindung der Formel II

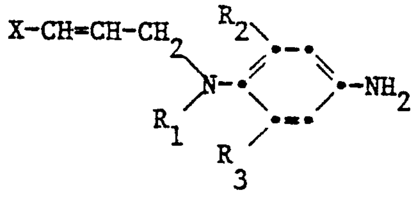

(II)

mit einer Verbindung der Formel III

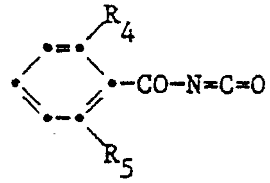

(III)

b) einer Verbindung der Formel IV

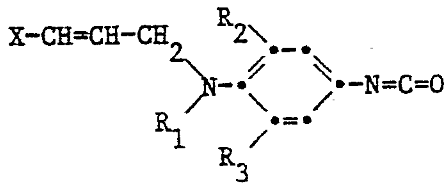

(IV)

mit einer Verbindung der Formel V

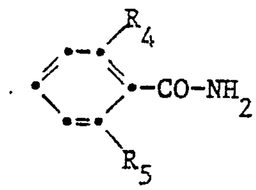

(V)

oder

c) einer Verbindung der Formel II mit einer Verbindung der Formel VI

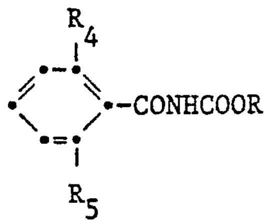

(VI)

In den obigen Formeln II, III, IV, IV, V und VI haben die Reste $R_1$, $R_2$, $R_3$, $R_4$, $R_5$ und X die unter Formel I vorstehend angegebenen Bedeutungen und R bedeutet einen gegebenenfalls mit Halogen substituierten niederen oder mittleren $C_1$-$C_8$-Alkylrest.

Die erwähnten Verfahren a) bis c) können vorzugsweise unter normalem Druck und in Gegenwart eines organischen Lösungs- oder Verdünnungsmittels durchgeführt werden. Als Lösungs- oder Verdünnungsmittel eignen sich z. B. Aether und ätherartige Verbindungen, wie Diäthyläther, Dipropyläther, Dibutyläther, Dioxan, Dimethoxyäthan und Tetrahydrofuran ; N,N-dialkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid sowie Ketone, z. B. Aceton, Methyläthylketon, Methylisopropylketon und Methylisobutylketon. Verfahren a) wird im allgemeinen bei einer Temperatur von − 10 bis 100 °C, vorzugsweise zwischen 15 und 25 °C, gegebenenfalls in Gegenwart einer organischen Base, z. B. Triäthylamin, durchgeführt. Die Durchführung von Verfahren b) erfolgt bei einer Temperatur von 0 bis 150 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Für die Umsetzung der Urethane VI gemäss Verfahren c) wählt man Temperaturen zwischen 60 °C und dem Siedepunkt des Reaktionsgemisches. Als Lösungsmittel eignen sich vor allem aromatische Kohlenwasserstoffe, wie Toluol, Xylol, Chlorbenzol usw.

Die Herstellung der Salze der Verbindung der Formel I erfolgt nach allgemein bekannten Arbeitsweisen.

Die Ausgangsstoffe der vorstehenden Formeln II, III, IV, V und VI sind bekannt oder lassen sich, falls sie neu sind, analog bekannten Verfahren herstellen. So können z. B. die p-Phenylendiamine der Formel II durch N-Halo-alkenylierung und gegebenenfalls N-Alkylierung der entsprechenden p-Nitroaniline und nachfolgende chemische Reduktion (z. B. mit Eisen in wässrigen Säuren) der Nitrogruppe zur Aminogruppe hergestellt werden. Eine weitere Möglichkeit zur Herstellung der p-Phenylendiamine der Formel II besteht in der Umsetzung des p-Halogennitrobenzols der Formel VII

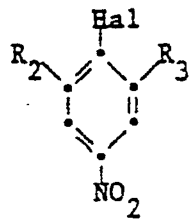

(VII)

mit einem sekundären Amin der Formel VIII

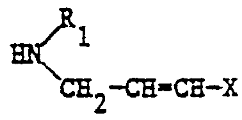

(VIII)

und anschliessende Reduktion der Nitrogruppe, wie vorstehend angegeben, wobei, $R_1$, $R_2$ und $R_3$ die oben genannten Bedeutungen haben und Hal ein Halogenatom, vorzugsweise Chlor oder Brom, bedeutet. Die Isocyanate der Formel IV sind durch Phosgenisierung der entsprechenden N,N-substituierten p-Phenylendiamine der Formel II (oder ihrer Hydrochloride) nach allgemein üblichen Arbeitsweisen erhältlich. Zu den Verbindungen der Formel III kann man wie folgt gelangen (vgl. J. Agr. Food Chem. *21(3)*, 348 bzw. 993 ; 1973) :

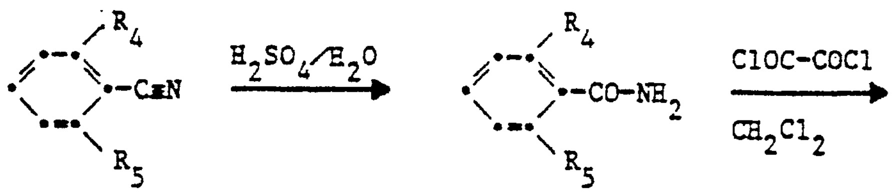


(III)

(IX) (X)

In vorstehend angegebenen Formeln haben $R_4$ und $R_5$ die unter Formel I angegebenen Bedeutungen. Zu den Verbindungen der Formel VI gelangt man durch Umsatz der Benzamide X mit entsprechenden Chlorameisensäureestern.

Es ist bereits bekannt, dass bestimmte N-Phenyl-N'-benzoylharnstoffe insektizide Eigenschaften besitzen (vgl. deutsche Offenlegungsschriften 2.123.236, 2.504.982, 2.537.413, 2.601.780 und 2.726.684, die belgischen Patentschriften 832.304, 843.906, 844.066 und 867.046 sowie die US-Patentschrift 4.089.975). Die deutsche Offenlegungsschrift 2.926.480 hat substituierte N-(p-Alkylenphenylamino)-phenyl-N'-benzylharnstoffe und -thioharnstoffe mit insektizider Wirksamkeit zum Gegenstand. Aus J. Agr. Food Chem. *21*, N° 3, 348ff. (1973) sind weiterhin substituierte N-Phenyl-N'-2,6-dichlorbenzoylharnstoffe bekannt, die insektizide Eigenschaften aufweisen sollen. Auf Seite 353 dieser Veröffentlichung werden entsprechende N-(4-Dimethylamino)-phenyl- und N-(3-Chlor-4-dimethylamino)-phenylderivate erwähnt, die jedoch — wie aus der dort aufgeführten Tabelle III ersichtlich — nur unzureichende Insektizidwirkung zeigen.

Uebarraschenderweise wurde demgegenüber gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenen Schädlingen.

Insbesondere eignen sich die Verbindungen der Formel I und die sie enthaltenden Mittel zur Bekämpfung von Insekten der Ordnungen : Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben ihrer sehr güngstigen Wirkung gegenüber Fliegen, wie z. B. Musca domestica, und Mückenlarven eignen sich Verbindungen der Formel I und die sie enthaltenden Mittel auch zur Bekämpfung von pflanzenschädigenden Frassinsekten, in Zier- und Nutzpflanzungen, insbesondere in Baumwollkulturen (z. B. gegen Spodoptera littoralis und Heliothis virescens) sowie in Obst- und Gemüsekulturen (z. B. gegen Leptinotarsa decemlineata, Pieris brassicae und Laspeyresia pomonella). Hervorzuheben ist besonders die ovizide und larvizide Wirkung von Verbindungen der Formel I. Werden Verbindungen der Formel I von adulten Insekten mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I und die sie enthaltenden Mittel eignen sich weiterhin zur Bekämpfung von Ektoparasiten, insbesondere von ektoparasitären Insekten, wie z. B. Lucilia sericata, an Haus- und Nutztieren, z. B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z. B. folgende Wirkstoffe in Betracht :

organische Phosphorverbindungen,
Nitrophenole und Derivate

Formamidine, Harnstoffe, Pyrethroide,
Carbamate, chlorierte Kohlenwasserstoffe und Bacillus thuringiensis-Präparate.

Es hat sich gezeigt, dass Präparate, die die erfindungsgemässen Verbindungen der Formel I zusammen mit dem bekannten Insektizid « Galecron » [$N^2$-(4-Chlor-2-methyl-phenyl)-$N^1$,$N^1$-dimethylformamidin] enthalten, sich durch überraschend hohe insektizide Wirksamkeit und ein besonders breites Anwendungs-Spektrum auszeichnen.

Mit besonderem Vorteil kann man die Verbindungen der Formel I auch mit Substanzen kombinieren, welche einen pestizid verstärkenden Effekt ausüben. Beispiele solcher Verbindungen sind u. a. : Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithiote.

Die Verbindungen der Formel I, bzw. deren Salze, werden als soche oder vorzugsweise zusammen mit den in der Formulierungstechnik üblichen Hilfsmitteln eingesetzt ; sie können z. B. zu Emulsionskonzentraten, direckt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in an sich bekannter Weise verarbeitet werden. Die Anwendungsverfahren, wie Versprühen, Vernebeln, Verstäuben, Verstreuen oder Giessen werden — ebenso wie die verwendeten Mittel — den angestrebten Zielen und gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialinen, z. B. Calcit oder Sand, in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder als gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind. z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate, wie z. B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxyaddukte, Tribuylphenoxypolyäthoxyäthanol, Polyäthylenglykol und Octylphenoxypolyäthoxyäthanol erwähnt.

Ferner kommen auch Fettsäureester von Polyoxyäthylensorbitan, wie das Polyoxyäthylensorbitantrioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, wie z. B. Stearyltrimethylammoniumchlorid oder Benzyldi(2-chloräthyl)äthylammoniumbromid.

Die in der Formulierungstechnik gebräuchlichen Tenside sind u. a. in folgenden Publikationen beschrieben :

« McCutcheon's Detergents and Emulsifiers Annual » MC Publishing Corp., Ringwood, New Jersey, 1979.

Sisely and Wood, « Encyclopedia of Surface Active Agents », Chemical Publishing Co., Inc. New York.

Die pestiziden Zubereitungen enthalten in der Regel 0,01 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 %, eines Tensides.

Während als Handelsprodukte eher konzentrierte Mittel bevorzugt werden, verwendet der Endverbraucher in der Regel verdünnte Zubereitungen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für Wirkstoffe der Formel I (% = Gewichtsprozent)

| 1. Spritzpulver | a) | b) |
|---|---|---|
| Wirkstoff | 20 % | 60 % |
| Na-Ligninsulfonat | 5 % | 5 % |
| Na-Laurylsulfat | 3 % | — |
| Na-Diisobutylnaphthalinsulfonat | — | 6 % |
| Octylphenolpolyäthylenglykoläther (7-8 Mol AeO) | — | 2 % |
| Hochdisperse Kieselsäure | 5 % | 27 % |
| Kaolin | 67 % | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrat

| | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff | 5 % | 8 % |
| Talkum | 95 % | — |
| Kaolin | — | 92 % |

Man erhält anwendungsfertige Stäubemittel, indem der Wirkstoff mit den Träger vermischt und auf einer geeigneten Mühle vermahlen wird.

4. Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

5. Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Beispiel 1

5,2 g 3,5-Dichlor-4-N-methyl-N-(3-chlorprop-2-en-1-yl)-amino-anilin wurden in absolutem Aether gelöst und unter Kühlen und Feuchtigkeitsausschluss mit 4 g 2,6-Difluorbenzoylisocyanat versetzt. Der nach einiger Zeit ausgefallene Niederschlag wurde abgesaugt und aus Alkonol umkristallisiert. Man erhielt 5,6 g $N^1$-3,5-Dichlor-4-N-methyl-N-(3-chlorprop-2-en-1-yl)-amino-phenyl-$N^2$-2,6-difluorbenzoylharnstoff vom Schmelzpunkt 155-157 °C (Verbindung Nr. 1).

Analog den vorstehend beschriebenen Arbeitsweisen werden die folgenden Verbindungen der Formel I hergestellt :

(Siehe Tabellen Seite 8 ff.)

| Verbindung Nr. | $R_1$ | $R_2$ | $R_3$ | $R_4$ | $R_5$ | X | Schmelzpunkt [°C] |
|---|---|---|---|---|---|---|---|
| 1 | $CH_3$ | Cl | Cl | F | F | Cl | 155 - 157 |
| 2 | $CH_3$ | H | H | F | F | Cl | 163 - 171 |
| 3 | $-(CH_2)_3-CH_3$ | Cl | Cl | F | F | Cl | 115 - 118 |
| 4 | $-CH_2-CH=CHCl$ | Cl | Cl | F | F | Cl | 160 - 162 |
| 5 | $-CH(CH_3)_2$ | Cl | Cl | F | F | Cl | 152 - 154 |
| 6 | $-(CH_2)_3-CH_3$ | Cl | Cl | F | H | Cl | 88 - 90 |
| 7 | $-CH_2-CH=CH_2$ | Cl | Cl | F | F | Cl | 98 - 100 |
| 8 | $-CH_3$ | Cl | Cl | Cl | H | Cl | 163 - 165 |
| 9 | $-CH_3$ | Cl | Cl | F | Cl | Cl | 167 - 168 |
| 10 | $-CH_3$ | Cl | Cl | F | H | Cl | 163 - 165 |
| 11 | $-CH_3$ | Cl | Cl | Cl | Cl | Cl | |
| 12 | $-CH_3$ | Cl | Cl | Br | H | Cl | 169 - 171 |
| 13 | $-CH_3$ | Cl | Cl | $CH_3$ | H | Cl | 153 - 155 |
| 14 | $-CH_3$ | Cl | Br | F | F | Cl | 158 - 159 |
| 15 | $-CH_3$ | Cl | Br | Cl | H | Cl | 167. - 169 |
| 16 | $-CH_3$ | Br | Br | F | F | Cl | |
| 17 | $-CH_3$ | Br | Br | Cl | H | Cl | |
| 18 | 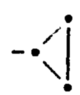 | Cl | Cl | F | F | Cl | |

| Verbindung Nr. | $R_1$ | $R_2$ | $R_3$ | $R_4$ | $R_5$ | X | Schmelzpunkt [°C] |
|---|---|---|---|---|---|---|---|
| 19 | 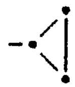 | Cl | Cl | Cl | H | Cl | 123 - 124 |
| 20 | 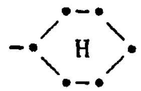 | Cl | Cl | F | F | Cl | |
| 21 | 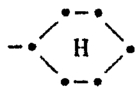 | Cl | Cl | Cl | H | Cl | |
| 22 | $-CH_3$ | H | H | Cl | H | Cl | |
| 23 | $-CH_3$ | H | H | F | H | Cl | |
| 24 | $-CH_2-CH=CHCl$ | H | H | F | F | Cl | |
| 25 | H | Cl | Cl | F | F | Cl | |
| 26 | $CH_3$ | Cl | Cl | F | F | Br | 139 - 141 |
| 27 | $CH_3$ | Cl | Cl | Cl | F | Br | 147 - 148 |
| 28 | $CH_3$ | Cl | Cl | Cl | H | Br | 149 - 151 |
| 29 | $CH_3$ | Cl | Cl | F | H | Br | 151 - 154 |
| 30 | $CH_3$ | Cl | Cl | Cl | Cl | Br | |
| 31 | $CH_3$ | Cl | Cl | $CH_3$ | H | Br | 145 - 147 |
| 32 | $CH_3$ | Br | Cl | F | F | Br | 145 - 147 |
| 33 | $CH_3$ | Br | Cl | Cl | H | Br | 155 - 156,5 |

| Verbindung Nr. | $R_1$ | $R_2$ | $R_3$ | $R_4$ | $R_5$ | X | Schmelzpunkt [°C] |
|---|---|---|---|---|---|---|---|
| 34 | $CH_3$ | Br | Br | F | F | Br | |
| 35 | $CH_3$ | Br | Br | Cl | H | Br | |
| 36 | $CH_3$ | Cl | Cl | Br | H | Br | 159 - 161 |
| 37 | $-CH_2-CH=CHBr$ | Cl | Cl | F | F | Br | |
| 38 | $-CH_2-CH=CHBr$ | Cl | Cl | Cl | H | Br | |
| 39 | $CH_3$ | Cl | Br | Cl | F | Br | 159 - 160 |
| 40 | $CH_3$ | Cl | Br | F | H | Br | 158 - 160 |

Beispiel 2

Wirkung gegen Lucilia sericata :

Zu 9 ml eines Zuchtmediums wurde bei 50 °C 1 ml einer 0,5 % Aktivsubstanz enthaltenden wässrigen Zubereitung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss Beispiel 1 zeiten in diesem Test gute Wirkung gegen Lucilia sericata.

Beispiel 3

Wirkung gegen Aëdes aegypti :

Auf die Oberfläche von 150 ml Wasser, das sich in einem Behälter befindet, wurde so viel einer 0,1 %igen acetonischen Lösung des Wirkstoffes pipettiert, dass Konzentrationen von je 10, 5 und 1 ppm erhalten wurden. Nach Verdunsten des Acetons wurde der Behälter mit 30-40 3-tägigen Aëdes-Larven beschickt. Nach 1, 2 und 5 Tagen wurde die Mortalität geprüft.

Verbindungen gemäss Beispiel 1 zeigten in diesem Test gute Wirkung gegen Aëdes aegypti.

Beispiel 4

Insektizide Frassgift-Wirkung

Baumwollpflanzen wurden mit wässrigen Wirkstoffemulsionen (erhalten aus einem 10 %igen emulgierbaren Konzentrat) besprüht, die ansteigende Mengen der zu prüfenden Wirkstoff-Verbindung enthielten.

Nach dem Antrocknen des Belages wurden die Baumwollpflanzen je mit Spodoptera littoralis- und Heliothis virecens-Larven im dritten larvalen Stadium besetzt. Der Versuch wurde bei 24 °C und 60 % relativer Luftfeuchtigkeit durchgeführt.

Die Auswertung auf prozentuale Abtötung und Frasschaden der Test-Insekten erfolgte in Abständen von jeweils 24 Stunden.

Beispiel 5

Ovizide Wirkung auf Spodoptera littoralis :

Auf Filterpapier abgelegte Eier von Spodoptera littoralis, die nicht älter als 24 Stunden waren, wurden aus dem Papier ausgeschnitten und in eine Lösung von 400 ppm des Wirkstoffes in einem Aceton-Wasser-Gemisch (1 : 1) getaucht. Die Eintauchzeit betrug eine Minute. Die so behandelten Eiablagen wurden dann aus der Lösung herausgenommen und bei 28 °C und 60 % relativer Feuchtigkeit in Kunstoffschalen deponiert.

Nach 5 Tagen wurde die Schlupfrate, d. h. die Anzahl Larven, die sich aus den behandelten Eiern entwickelt hatten, bestimmt.

Verbindungen gemäss Beispiel 1 zeigten gute Wirkung im obigen Test.

Beispiel 6

Wirkung auf Laspeyresia pomonella (Eier) :

Abgelegte Eier von Laspeyresia pomonella, die nicht älter als 24 Stunden waren, wurden auf Filterpapier für 1 Minute in acetonischwässrige Lösungen enthaltend ansteigende Mengen des zu prüfenden Wirkstoffes eingetaucht. Nach dem Antrocknen der Lösung wurden die Eier in Petrischalen ausgelegt und bei einer Temperatur von 28 °C belassen. Nach 6 Tagen wurde der prozentuale Schlupf aus den behandelten Eiern bewertet.

Beispiel 7

Chemosterilisierende Wirkung auf Anthonomus grandis.

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und

Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z. B. « Actamer B 100 ») desinfiziert und dann in Schalen, die eine geeignete Larvalidiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss Beispiel 1 zeigten gute Wirkung im obligem Test.

## Biologische Ergebnisse

Die nachstehende Tabelle zeigt Ergebnisse biologischer Prüfungen erfindungsgemässer Verbindungen auf der Basis der obigen biologischen Beispiele. Die Auswertung der Versuche erfolgte anhand der erhaltenen %-Mortalität unter Verwendung des folgenden Bewertungs-Index :

A : 80-100 % Mortalität bei einer Konzentration von 0,75 ppm der geprüften Verbindung.
B : 80-100 % Mortalität bei einer Konzentration von 3,0 ppm der geprüften Verbindung.
C : 80-100 % Mortalität bei einer Konzentration von 12,5 ppm der geprüften Verbindung.
D : 80-100 % Mortalität bei einer Konzentration von 50 ppm der geprüften Verbindung.
E : 80-100 % Mortalität bei einer Konzentration von 100 ppm der geprüften Verbindung.
F : 80-100 % Mortalität bei einer Konzentration von 200 ppm der geprüften Verbindung.
G : 80-100 % Mortalität bei einer Konzentration von 400 ppm der geprüften Verbindung.
H : weniger als 80 % Mortalität bei einer Konzentration von 800 ppm der geprüften Verbindung
— : nicht geprüft.

| Verbindung | pestizide Wirksamkeit | | |
|---|---|---|---|
| Nr. | Spodoptera (Beispiel 4) | Heliothis (Beispiel 4) | Laspeyresia (Beispiel 6) |
| 1 | A | C | C |
| 2 | H | H | - |
| 3 | B | D | - |
| 4 | C | H | - |
| 5 | C | E | - |
| 6 | A | D | - |
| 7 | C | D | - |
| 8 | D | G | C |
| 9 | D | F | F |
| 10 | C | D | - |
| 12 | D | G | - |
| 13 | C | F | E |
| 14 | D | D | C |
| 15 | D | F | D |
| 19 | B | D | - |
| 26 | B | C | D |
| 27 | B | D | F |

(Fortsetzung)

| Verbindung | pestizide Wirksamkeit | | |
|---|---|---|---|
| Nr. | Spodoptera (Beispiel 4) | Heliothis (Beispiel 4) | Laspeyresia (Beispiel 6) |
| 28 | C | E | C |
| 29 | D | E | - |
| 31 | C | G | - |
| 32 | C | D | C |
| 36 | D | G | - |

**Ansprüche**

1. Verbindungen der Formel I

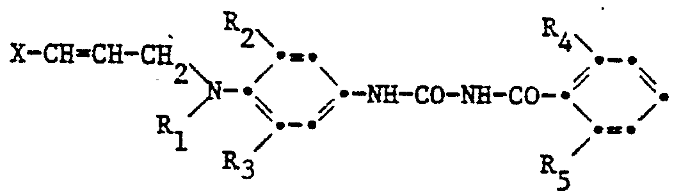

(I)

worin $R_1$ Wasserstoff, $C_1$-$C_4$-Alkyl, $C_3$-Alkenyl, mit einem Chlor- oder Bromatom substituiertes $C_3$-Alkenyl oder $C_3$-$C_6$-Cycloalkyl ; $R_2$ und $R_3$ Wasserstoff oder Halogen ; $R_4$ Methyl oder Halogen, $R_5$ Wasserstoff oder Halogen und X Chlor oder Brom bedeuten, sowie ihre Salze.

2. Verbindungen der Formel I gemäss Anspruch 1, dadurch gekennzeichnet, dass $R_1$ $C_1$-$C_4$-Alkyl, $CH_2 = CH—CH_2—$, $Cl—CH = CH—CH_2—$, $Br—CH = CH—CH_2—$ oder Cyclopropyl ; $R_2$ und $R_3$ Chlor oder Brom ; $R_4$ Fluor, Chlor, Brom oder Methyl, $R_5$ Wasserstoff, Fluor oder Chlor und X Chlor oder Brom bedeuten.

3. Verbindungen der Formel I gemäss Anspruch 2, dadurch gekennzeichnet, dass $R_1$ $C_1$-$C_4$-Alkyl ; $R_2$ und $R_3$ Chlor oder Brom, $R_4$ Fluor oder Chlor, $R_5$ Wasserstoff, Fluor oder Chlor und X Chlor oder Brom bedeuten.

4. Verbindung der Formel I gemäss den Ansprüchen 1 bis 3, dadurch gekennzeichnet, dass $R_2$ und $R_3$ Chlor bedeuten.

5. Verbindung der Formel I gemäss den Ansprüchen 1 bis 4, dadurch gekennzeichnet, dass $R_1$ Methyl bedeuten.

6. Verbindung der Formel I gemäss den Ansprüchen 1 bis 5, dadurch gekennzeichnet, dass $R_4$ und $R_5$ Fluor oder Chlor bedeuten.

7. Verbindung der Formel I gemäss Anspruch 6, dadurch gekennzeichnet, dass $R_4$ und $R_5$ Fluor bedeuten.

8. Verbindung gemäss Anspruch 3 der Formel

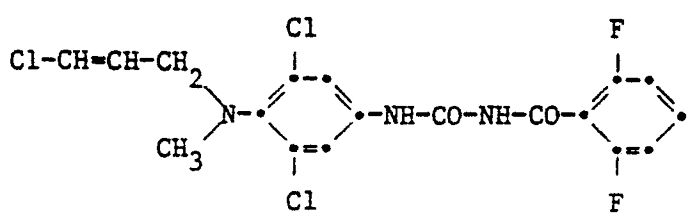

9. Verbindung gemäss Anspruch 3 der Formel

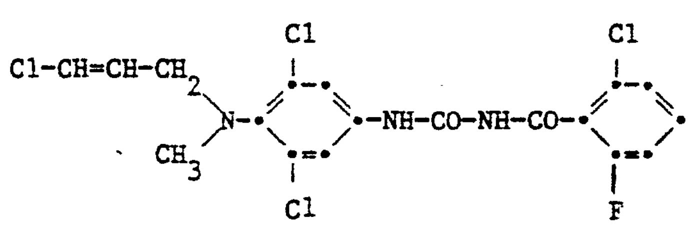

10. Verbindung gemäss Anspruch 3 der Formel

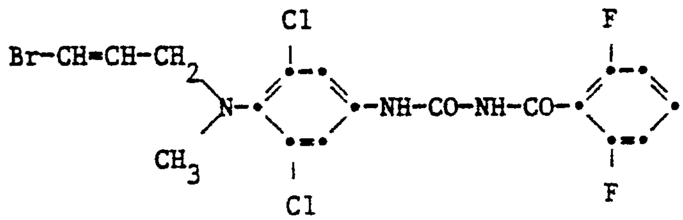

11. Verbindung gemäss Anspruch 3 der Formel

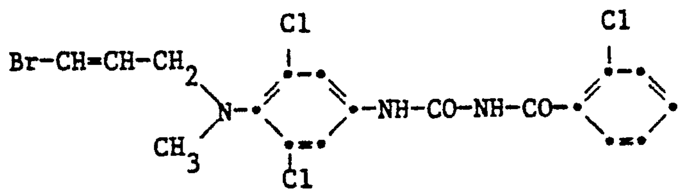

12. Verfahren zur Herstellung einer Verbindung der Formel I gemäss den Ansprüchen 1 bis 11, dadurch gekennzeichnet, dass man

a) eine Verbindung der Formel II

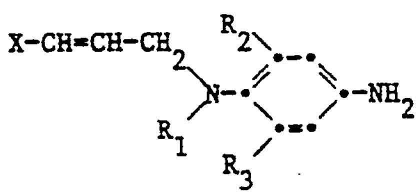

(II)

mit einer Verbindung der Formel III

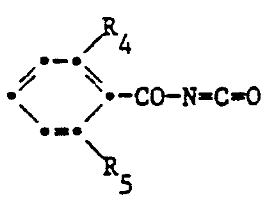

(III)

b) eine Verbindung der Formel IV

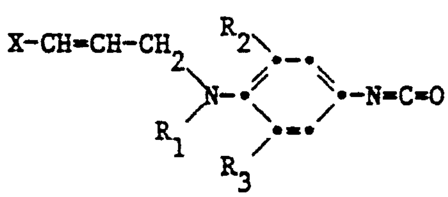

(IV)

mit einer Verbindung der Formel V

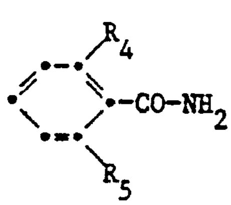

(V)

oder

c) eine Verbindung der Formel II mit einer Verbindung der Formel VI

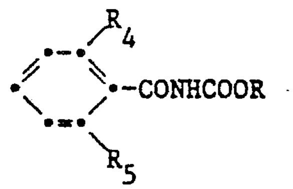

(VI)

umsetzt, wobei in den Formeln II bis VI die Reste $R_1$ bis $R_5$ und X die in den Ansprüchen 1 bis 7 angegebenen Bedeutungen haben, und R eine gegebenenfalls mit Halogen substituierte $C_1$-$C_8$ Alkylgruppe bedeutet, und dass man die erhaltene Verbindung der Formel I gegebenenfalls mit einer Säure in ein Salz überführt.

13. Schädlingsbekämpfungsmittel, welches als aktives Komponente eine Verbindung gemäss den Ansprüchen 1 bis 11 zusammen mit geeigneten Trägern und/oder anderen Zuschlagstoffen enthält.

14. Verwendung von Verbindungen gemäss den Ansprüchen 1bis 11 zur Bekämpfung von Schädlingen, vorzugsweise von Insekten, insbesondere von pflanzenschädigenden Insekten.

**Claims**

1. A compound of the formula I

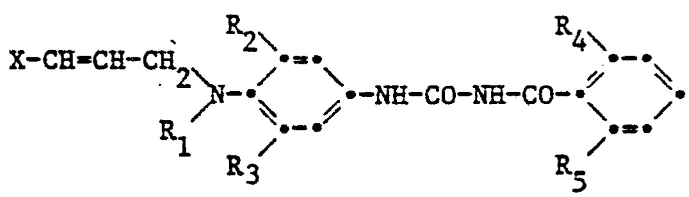

(I)

wherein $R_1$ is hydrogen, $C_1$-$C_4$-alkyl, $C_3$-alkenyl, $C_3$-alkenyl substituted by a chlorine or bromine atom, or $R_1$ is $C_3$-$C_6$-cycloalkyl, $R_2$ and $R_3$ are hydrogen or halogen, $R_4$ is methyl or halogen, $R_5$ is hydrogen or halogen, and X is chlorine or bromine ; and salts thereof.

2. A compound of the formula I according to Claim 1, wherein $R_1$ is $C_1$-$C_4$-alkyl, $CH_2=CH—CH_2—$, $Cl—CH=CH—CH_2—$, $Br—CH=CH—CH_2—$ or cyclopropyl, $R_2$ and $R_3$ are chlorine or bromine, $R_4$ is fluorine, chlorine, bromine or methyl, $R_5$ is hydrogen, fluorine or chlorine, and X is chlorine or bromine.

3. A compound of the formula I according to Claim 2, wherein $R_1$ is $C_1$-$C_4$-alkyl, $R_2$ and $R_3$ are chlorine or bromine, $R_4$ is fluorine or chlorine, $R_5$ is hydrogen, fluorine or chlorine, and X is chlorine or bromine.

4. A compound of the formula I according to Claims 1 to 3, wherein $R_2$ and $R_3$ are chlorine.

5. A compound of the formula I according to Claims 1 to 4, wherein $R_1$ is methyl.

6. A compound of the formula I according to Claims 1 to 5, wherein $R_4$ and $R_5$ are fluorine or chlorine.

7. A compound of the formula I according to Claim 6, wherein $R_4$ and $R_5$ are fluorine.

8. A compound according to Claim 3 of the formula

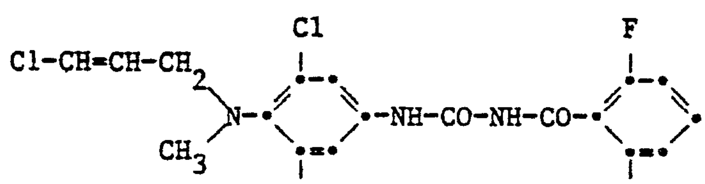

9. A compound according to Claim 3 of the formula

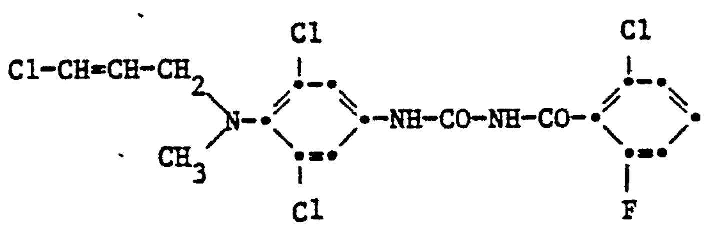

10. A compound according to Claim 3 of the formula

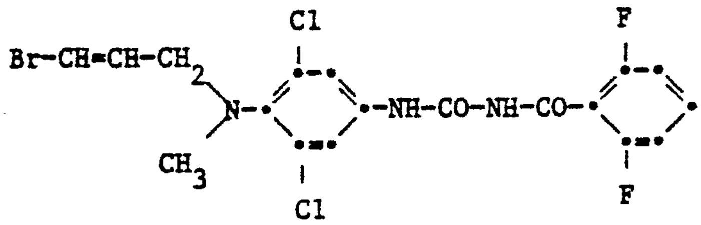

11. A compound according to Claim 3 of the formula

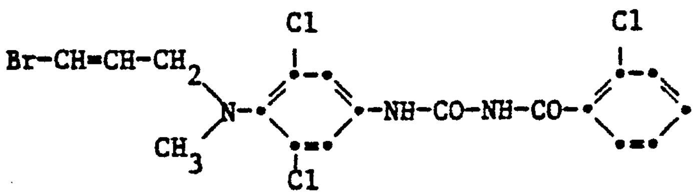

12. A process for producing a compound of the formula I according to Claims 1 to 11, which process comprises

a) reacting a compound of the formula II

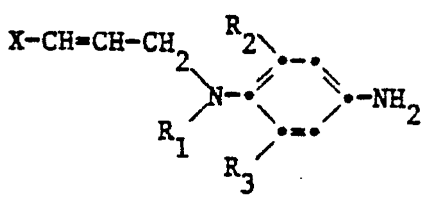

(II)

with a compound of the formula III

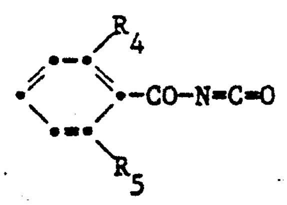

(III)

or

b) reacting a compound of the formula IV

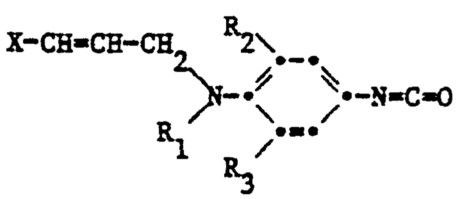

(IV)

with a compound of the formula V

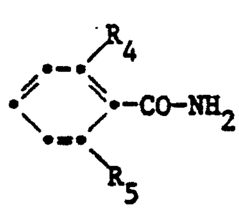

(V)

or

c) reacting a compound of the formula II with a compound of the formula VI

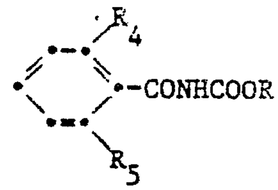

(VI)

the symbols $R_1$ to $R_5$ and X in the formulae II to VI having the meanings defined in Claims 1 to 7, and R being a $C_1$-$C_8$-alkyl group which is unsubstituted or substituted by halogen ; and optionally converting the resulting compound of the formula I with an acid into a salt.

13. A pesticidal composition which contains as active ingredient a compound according to Claims 1 to 11, together with suitable carriers and/or other additives.

14. A method of combating pests at a locus, in particular insects, especially insects which damage plants, which method comprises applying to the said locus an insecticidally effective amount of a compound as claimed in any one of Claims 1 to 11.

**Revendications**

1. Composés de formule I

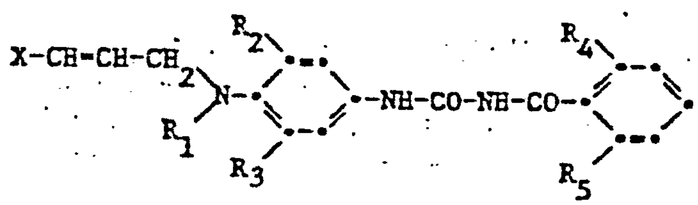

(I)

dans laquelle $R_1$ représente de l'hydrogène, un alkyle en $C_1$-$C_4$, un alcényle en $C_3$, un alcényle en $C_3$ ou un cycloalkyle en $C_3$ à $C_6$ substitué par un atome de chlore ou de brome ; $R_2$ et $R_3$ représentent de l'hydrogène ou un halogène ; $R_4$ représente un méthyle ou un halogène, $R_5$ représente de l'hydrogène ou un halogène et X représente du chlore ou du brome, ainsi que leurs sels.

2. Composés de formule I selon la revendication 1, caractérisés en ce que $R_1$ représente un alkyle en $C_1$ à $C_4$, $CH_2=CH—CH_2—$, $Cl—CH=CH—CH_2—$, $Br—CH=CH—CH_2—$ ou un cyclopropyle ; $R_2$ et $R_3$ représentent du chlore ou du brome ; $R_4$ représente du fluor, du chlore, du brome ou un méthyle, $R_5$ représente de l'hydrogène, du fluor ou du chlore et X représente du chlore ou du brome.

3. Composés de formule I selon la revendication 2, caractérisés en ce que $R_1$ représente un groupe alkyle en $C_1$ à $C_4$ ; $R_2$ et $R_3$ du chlore ou du brome, $R_4$ du fluor ou du chlore, $R_5$ de l'hydrogène, du fluor ou du chlore et X du chlore ou du brome.

4. Composé de formule I selon l'une des revendications 1 à 3, caractérisé en ce que $R_2$ et $R_3$ représentent du chlore.

5. Composé de formule I selon l'une des revendications 1 à 4, caractérisé en ce que $R_1$ représente un méthyle.

6. Composé de formule I selon l'une des revendications 1 à 5, caractérisé en ce que $R_4$ et $R_5$ représentent du fluor ou du chlore.

7. Composé de formule I selon la revendication 6, caractérisé en ce que $R_4$ et $R_5$ représentent du fluor.

8. Composé selon la revendication 3 de formule

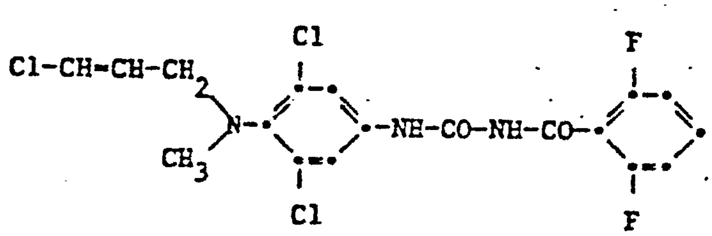

9. Composé selon la revendication 3 de formule

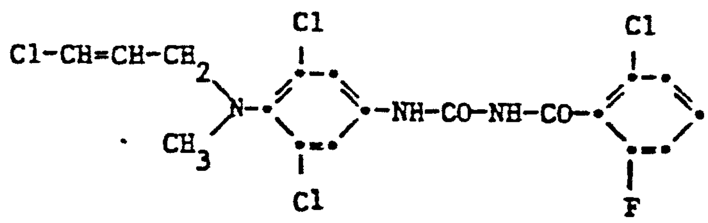

10. Composé selon la revendication 3 de formule

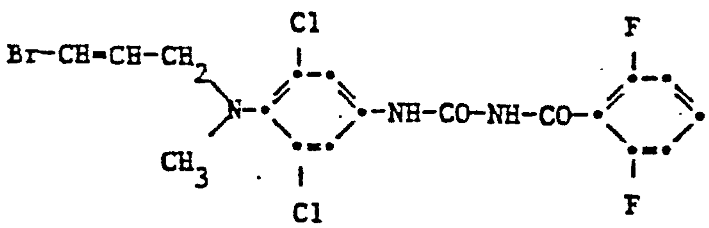

11. Composé selon la revendication 3 de formule

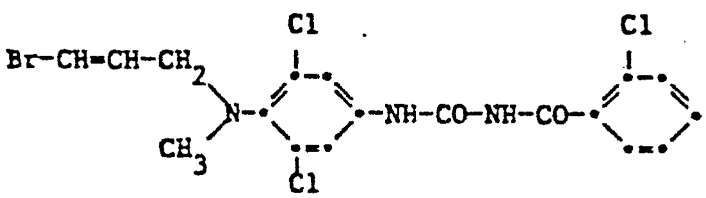

12. Procédé de préparation d'un composé de formule I selon l'une des revendications 1 à 11, caractérisé en ce que l'on fait réagir

a) un composé de formule II

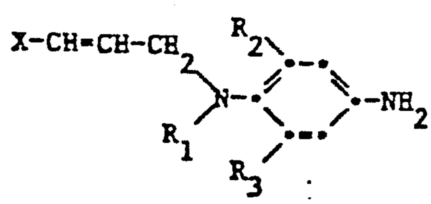

(II)

avec un composé de formule III

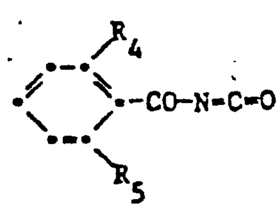

(III)

b) un composé de formule IV

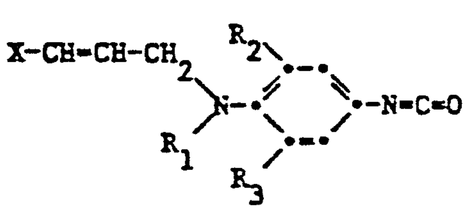

(IV)

avec un composé de formule V

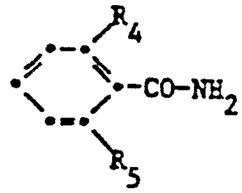

(V)

ou bien

c) un composé de formule II avec un composé de formule VI

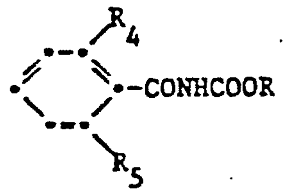

(VI)

dans les formules II à VI, les radicaux $R_1$ à $R_5$ et X ayant les significations données dans les revendications 1 à 7, et R représentant un groupe alkyle en $C_1$ à $C_8$ éventuellement substitué par un halogène, et en ce que l'on fait réagir éventuellement le composé de formule I obtenu avec un acide pour donner un sel.

13. Agent de lutte contre les parasites, contenant comme substance active un composé selon l'une des revendications 1 à 11 ainsi que des supports ou autres additifs appropriés.

14. Utilisation de composés selon l'une des revendications 1 à 11, dans la lutte contre les parasites de préférence des insectes, en particulier, des insectes nuisibles pour les plantes.